# EUROPÄISCHE PATENTSCHRIFT

(11) **EP 1 658 299 B1**
(45) Veröffentlichungstag und Bekanntmachung des Hinweises auf die Patenterteilung: **23.09.2009**
(21) Anmeldenummer: 04740776.2
(22) Anmeldetag: 08.07.2004
(51) Int. Cl.: C07F 9/6571

(54) **VERFAHREN ZUR HERSTELLUNG VON CYCLISCHEN PHOSPHONS UREANHYDR IDEN**
METHOD FOR THE PRODUCTION OF CYCLIC PHOSPHONIC ACID ANHYDRIDES
PROCEDE DE PRODUCTION D'ANHYDRIDES D'ACIDE PHOSPHONIQUE CYCLIQUES

(30) Priorität: 21.07.2003 DE 10333042
(43) Veröffentlichungstag der Anmeldung: 24.05.2006
(73) Patentinhaber: Archimica GmbH, 65929 Frankfurt am Main (DE)
(72) Erfinder: WEHNER, Mark, 65527 Niedernhausen (DE); KIRSCHBAUM, Bettina, 2300 Turnhout (BE); DEUTSCHER, Lothar, 65824 Schwalbach (DE); WAGNER, Hans, Jürgen, 65795 Hattersheim (DE); HÖSSL, Harald, 65779 Kelkheim (DE)
(74) Vertreter: Schweitzer, Klaus
(86) Internationale Anmeldenummer: PCT/EP2004/007468
(87) Internationale Veröffentlichungsnummer: WO 2005/014604

(56) Entgegenhaltungen:
- SIDNEY H METZGER ET AL: "Highly Branched Alkylphosphorous Compounds. II. Synthesis of 1,1,2-Trimethylpropylphosphonyl Chloride" JOURNAL OF ORGANIC CHEMISTRY, Bd. 29, Nr. 3, 1964, Seiten 627-630, XP002316581
- WISSMANN H ET AL: "New peptide synthesis" ANGEWANDTE CHEMIE. INTERNATIONAL EDITION, VERLAG CHEMIE. WEINHEIM, DE, Bd. 19, Nr. 2, 1980, Seiten 133-134, XP002115465 ISSN: 0570-0833 in der Anmeldung erwähnt
- DIEMERT, KLAUS ET AL: "A convenient synthesis of phosphonic anhydrides. Trimers [RPO2]3 (R = tert-butyl, 2-methylphenyl, 2,4,6-trimethylphenyl). Their structures and reaction products" EUROPEAN JOURNAL OF INORGANIC CHEMISTRY , (3), 361-366 CODEN: EJICFO; ISSN: 1434-1948, 1998, XP002316582

## Beschreibung

Die vorliegende Erfindung betrifft einen verbessertes Verfahren zur Herstellung von bekannten 2,4,6-substituierten 1,3,5,2,4,6-trioxatriphosphinan-2,4,6-trioxiden (III) aus den zugrundeliegenden Phosphonsäuren der Formel (I) über ihre offenkettigen Analoga der Formel (II), durch Destillation.

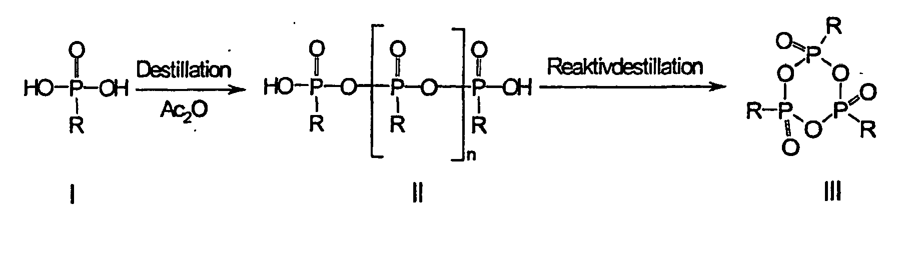

Die Herstellung der oligomeren Phosphonsäureanhydride der Formel (II) (OPA) durch Kondensation mit einem geeigneten Hilfsmittel (EP-B-0 527 442) und die Verwendung dieser OPA zur Bildung von Amidbindungen (DE-A-38 39 379) ist bereits bekannt.

Allerdings müssen hier zur Knüpfung von Amidbindungen entsprechend dem Stand der Technik Überschüsse der OPA eingesetzt werden, weil in dem nach EP 0 527 442 hergestellten Produkt Phosphonsäureanhydride der Kettenlänge P₂₀ - P₂₀₀ hergestellt werden und somit die Zusammensetzung der OPA nicht genau bekannt ist. Dadurch bedingte Kosten können durch die mit dem erfindungsgemäßen Verfahren hergestellten cyclichen Propanphosphonsäureanhydride der Formel (III) (CPA) vermieden werden.

Phosphonsäuren werden von Mikroorganismen üblicherweise zum Phosphat abgebaut welches ökolgisch problematisch ist (Eutrophierung). Durch den verminderten, weil stöchiometrischen Einsatz der cyclischen Phosphonsäureanhydriden (CPA) kann dies vermieden werden. Dadurch ist ein weiterer Vorteil erzielt durch die Herstellung und den Einsatz von Verbindungen der Formel (III) gegenüber den bekannten Herstellungsverfahren und Anwendungen wie beschrieben in EP-A-0 527 442 und EP-A-3 839 379.

Die gezielte Herstellung von CPA's ist im Labor bereits gelungen (H. Wissmann, H. J. Kleiner, Angew. Chem. Int. Ed. 1980, 19, 133 [129]). Die Möglichkeit einer Destillation der CPA's wird hier bereits erwähnt, dabei handelt es sich aber um eine zusätzliche Reindestillation von bereits als Rohprodukten vorliegenden CPA's. Außerdem wurden bei diesem Verfahren die CPA's unter Verwendung von Phosphonsäuredichloriden hergestellt.

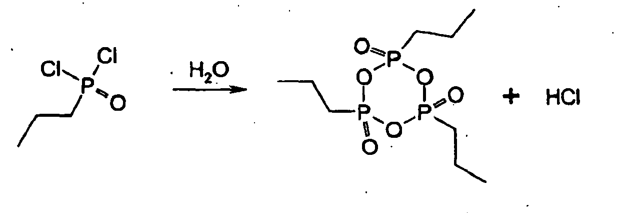

Das in diesem Herstellungsprozeß entstehende HCl wirft bei einer technischen Anwendung dieser Syntheseroute, durch seine korrosiven und giftigen Eigenschaften viele Probleme auf. Hinzu kommt, daß das Produkt Chlorid enthält, welches die Anwendungsmöglichkeiten der CPA's einschränken kann.

In SIDNEY H. METZGER ET AL: "Highly Branched Alkylphosphorous Compounds. II. Synthesis of 1,1,2-Trimethylpropylphosphonyl Chloride", JOURNAL OF ORGANIC CHEMISTRY, Bd. 29, Nr. 3,1964, S. 627-630, XP002316581, wird die Bildung von einem cyclischen Phsphonsäureanhydrid durch Umsetzung eines Phosphonsäurederivats mit Thionylchlorid beschrieben.

Im Hinblick auf die vorstehend erwähnten Nachteile der bekannten Verfahren, bei dem ein Produktgemisch in undefinierter Zusammensetzung erhalten wird, große Mengen Abfallstoffe anfallen, korrosive und giftige Gase entstehen oder das Produkt Chlorid enthält, besteht der Bedarf, ein verbessertes Verfahren bereitzustellen, das alle diese Nachteile nicht aufweist.

Diese Aufgabe wird gelöst durch ein Verfahren zur Herstellung von cyclischen Phosphonsäureanhydriden der Formel (III) durch
a) Umsetzung von Alkanphosphonsäuren der Formel (I) mit Essigsäureanhydrid bei einer Temperatur im Bereich von 30 bis 150°C und gleichzeitiger destillativer Abtrennung eines Gemisches aus Essigsäure und Essigsäureanhydrid,
b) anschließender Reaktivdestillation der in Schritt a) erhaltenen oligomeren Phosphonsäureanhydride der Formel (II) und Überführung in die entsprechenden cyclischen trimeren Phosphonsäureanhydride der Formel (III), 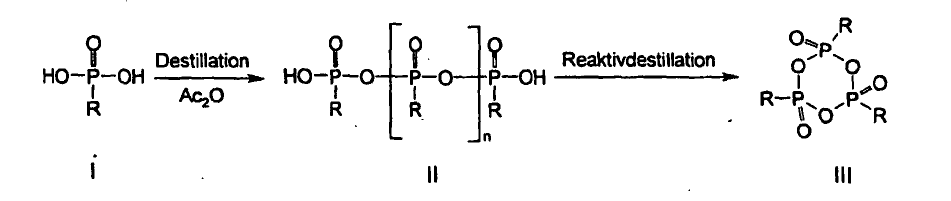
c) wobei die gebildeteten cyclischen trimeren Phosphonsäureanhydride sofort in einem organischen Lösungsmittel gelöst werden, das sich diesen gegenüber inert verhält
wobei,
n eine ganze Zahl von 0 bis 300 ist und
R für H, Fluor, Chlor, Brom, lod, Allyl, Aryl oder offenkettige cyclische oder verzweigte C₁ bis C₈-Alkyl-Reste, Aryloxy, Allyloxy oder Alkoxy mit offenkettigen cyclischen oder verzweigten C₁ bis C₈-Alkyl-Resten, Nitro, Nitril, Carboxy, Carbonsäureester mit offenkettigen oder verzweigten C₁ bis C₈-Alkyl-Resten, Amid- oder Alkylamidreste mit offenkettigen oder verzweigten C₁ bis C₈-AlkylResten steht.

Die in Schritt a) erhaltenenen OPA's der Formel (II) werden nach dem erfindungsgemäßen Verfahren, durch eine Reaktivdestillation direkt zu CPA's der Formel (III) umgesetzt und sofort nach Erhalt in einem geeigneten Lösungsmitteln gelöst. Auf diese Weise ist es möglich eine sofortige Polymerisation der CPAs unter Rückbildung der oligomeren Phosphonsäureanhydride zu vermeiden.

Geeignete Lösungsmittel sind jene, die nicht mit den Phosphonsäureanhydriden reagieren, wobei es sich insbesondere um aprotische organische Lösungsmittel handelt.

Als Lösungsmittel eignen sich alle aprotischen organischen Lösungsmittel, die nicht mit den CPA der Formel (III) reagieren, bevorzugt werden Ligroin, Pentan, Hexan, Heptan, Octan, Cyclopentan, Cyclohexan, Cycloheptan, Cyclooctan, Dichlormethan, Chloroform, Tetrachlorkohlenstoff, 1,2-Dichlorethan, 1,1,2,2-Tetrachlorethan, Methylacetat, Ethylacetat, Propylacetat, Buthylacetat, Dimethylformamid, Diethylformamid, Dimethylacetamid, Diethylacetamid, Diethylether, Diisopropylether, tert-Buthyl-Methylether, THF, Dioxan, Acetonitril, Sulfolan, DMSO, HMPT, NMP oder Gemische aus diesen, besonders bevorzugt sind Dichlormethan, Chloroform, Ethylacetat, Propylacetat, Buthylacetat, Dimethylformamid, Diethylformamid, Dimethylacetamid, Diethylacetamid, Diisopropylether, tert-Buthyl-Methylether, THF, Dioxan, Acetonitril oder Gemische aus diesen, ganz besonders bevorzugt werden Dichlormethan, Chloroform, Ethylacetat, Buthylacetat, Dimethylformamid, Dimethylacetamid, tert-Buthyl-Methylether, THF, Dioxan, Acetonitril oder Gemische aus diesen.
Während der Reaktivdestillation wird, nach der vollständigen destillativen Entfernung von Essigsäure und unumgesetztem Essigsäureanhydrid unter vermindertem Druck, das vorliegende OPA der Formel (II) (Herstellung analog EP-B-0 527 442) bei einem Vakuum von 0,001 mbar bis 500 mbar und einer Temperatur von 100°C bis 450°C gespalten und CPA's der Formel (III) rein erhalten.

Das erfindungsgemäße Verfahren zeichnet sich insbesondere dadurch aus, daß im Gegensatz zu den bisher bekannten Verfahren nur ein geringer apparativer Aufwand erforderlich ist, denn die Herstellung der OPA's der Formel (II) und die Reaktivdestillation zur Herstellung der CPA's der Formel (III) können im selben Reaktionsgefäß durchgeführt werden.
Es ist als sehr überraschend anzusehen, dass die Bildung der cyclischen Verbindungen (III) durch eine Reaktivdestillation gelingt. Durch die gewählten Bedingungen bei der Destillation kann das gewünschte CPA rein erhalten werden; eine Re-Oligomerisierung findet nicht statt.

Als Reste R in den Formeln (I), (II) und (III) sind wie vorstehend erwähnt Allyl, Aryl oder offenkettige cyclische oder verzweigte C₁ bis C₈-Alkyl-Reste, Aryloxy, Allyloxy oder Alkoxy mit offenkettigen cyclischen oder verzweigten C₁ bis C₈-Alkyl-Resten, geeignet. Besonders geeignet sind Reste mit R = Methyl, Ethyl, n-Propyl, Isopropyl, n-Butyl, 2-Butyl, Isobutyl, Pentyl, Hexyl, ganz besonders geeignet sind Ethyl-, Propyl- und Butyl-Reste.

Das Verhältnis von Essigsäureanhydrid zur der Phosphonsäure der Formel (I) kann beliebig gewählt werden, sollte aber nicht zu klein sein und liegt bevorzugt im Bereich zwischen 20 : 1 und 1 : 1, besonders bevorzugt zwischen 10 : 1 und 1 : 1 und ganz besonders bevorzugt zwischen 5 : 1 und 1 : 1.

Die Reaktion und Destillation bei dem erfindungsgemäßen Verfahren findet in zwei Stufen statt, und besteht
a) aus der Kondensation von Phosphonsäuren der Formel (I) zu OPA's der Formel (II) bei gleichzeitiger Destillation von Essigsäure und unumgesetztem Essigsäureanyhdrid in einem Temperaturbereich von 30°C bis 150°C (Reaktorinnentemperatur) bzw. von 30°C bis 130°C (Kopftemperatur) durchgeführt wird, bevorzugt jedoch im Temperaturbereich von 50°C bis 130°C (Reaktorinnentemperatur) bzw. von 35°C bis 100°C (Kopftemperatur), ganz besonders bevorzugt im Temperaturbereich von 70°C bis 110°C (Reaktorinnentemperatur) bzw. von 40°C bis 70°C (Kopftemperatur), und
b) aus der Reaktivdestillation der OPA der Formel (II) zu CPA der Formel (III) die im Temperaturbereich von 100°C und 450°C (Reaktorinnentemperatur) bzw. von 100°C bis 380°C (Kopftemperatur) erfolgt, bevorzugt jedoch im Temperaturbereich von 150°C und 400°C (Reaktorinnentemperatur) bzw. 150°C bis 350°C (Kopftemperatur), besonders bevorzugt im Temperaturbereich von 200 und 350°C (Reaktorinnentemperatur) bzw. 200°C bis 300°C (Kopftemperatur).

Entsprechend dem erfindungsgemäßen Verfahren liegt der Druck bei
a) der Destillation von Essigsäure und unumgesetztem Essigsäureanhydrid im Bereich von 1 mbar bis 1000 mbar, bevorzugt im Bereich von 10 mbar bis 500 mbar, besonders bevorzugt im Bereich von 50 mbar bis 200 mbar, und bei
b) der Reaktivdestillation der OPA der Formel (II) zu CPA der Formel (III) in einem Druckbereich von 0,001 mbar bis 500 mbar, bevorzugt im Bereich von 0,005 mbar bis 100 mbar, besonders bevorzugt im Bereich von 0,01 mbar bis 50 mbar.

Die Destillation kann über einen beliebigen Zeitraum erfolgen, häufig findet
a) die Destillation von Essigsäure und Essigsäureanhydrid jedoch innerhalb von 100 h, bevorzugt innerhalb von 80 h, besonders bevorzugt inerhalb von 60 h statt, und
b) die Reaktivdestillation von OPA der Formel (II) zu CPA der Formel (III) innerhalb von 120 h, bevorzugt innerhalb von 90 h, besonders bevorzugt innerhalb von 60 h statt.

Allgemein wurde beobachtet, daß die Destillation und die Reaktivdestillation in kleinen Maßstäben in einem kürzeren Zeitraum durchführbar sind, während sich die Reaktionszeiten beim Übergang z.B. zum Technikumsmaßstab erhöhen.

Das erhaltene CPA der Formel (III) wird sofort nach der Destillation in einem organischen Lösungsmittel gelöst, dabei kann das Mischungsverhältnis zwischen Lösungsmittel und CPA beliebig gewählt werden, sollte aufgrund der Viskosität der Verbindung jedoch nicht zu klein gewählt werden, und liegt bevorzugt im Bereich von 10 : 1 bis 1 : 10, besonders bevorzugt im Bereich von 5 : 1 bis 1 : 5, ganz besonders bevorzugt im Bereich von 2 : 1 bis 1 : 2. In einer bevorzugten Ausführungsform wird das CPA-Kondensat direkt in dem inerten organischen Lösungsmittel aufgefangen, um eine Polymerisation und Rückbildung zu den analogen Oligomeren zu verhindern.

Die so hergestellte Lösung aus CPA der Formel (III) und dem gewählten Lösungsmittel oder Lösungsmittelgemisch kann unmittelbar für Kondensationsreaktionen wie Amid- (M. Feigel et al., Angew. Chem. Int. Ed. 1989, 28, 486 [466]) und Esterknüpfungen (F.-P. Montforts et al., Eur. J. Org. Chem. 2001, 1681-1687), Acylierungen (DE 100 63 493) sowie zur Herstellung von Heterocyclen (WO 99/37620) eingesetzt werden.
Insbesondere besitzt das erfindungsgemäße Verfahren gegenüber dem Stand der Technik den Vorteil, daß aus den verschiedenen Oligomeren der Formel (II) eine Verbindung der Formel (III) von definierter Molmasse entsteht, welche dadurch in stöchiometrischen Mengen eingesetzt werden kann. Damit erhöht sich die Wirtschaftlichkeit der Anwendung dieser Phosphonsäureanhydride in chemischen Prozessen, wie z.B. in Kondensationsreaktionen von Carbonsäuren mit Alkoholen oder Aminen. Durch das Lösen der sirupösen Verbindung in einem organischen Lösungsmittel kann diese besonders leicht gehandhabt werden und ist in dieser Form überraschenderweise auch in eine Vielzahl von Reaktionen direkt einsetzbar.
Mit den so erhaltenen Lösungen können insbesondere Amidbindungen geknüpft und das Kopplungsreagenz in definierter und sparsamer Menge zugegeben werden.

Die nachfolgenden Beispiele und Vergleichsbeispiele dienen zur Veranschaulichung des Gegenstands der Erfindung, ohne dass die Erfindung auf diese Beispiele beschränkt sein soll.

### Beispiele:

### Beispiel 1: Synthese von 2,4,6-Tripropyl-[1,3,5,2,4,6]-trioxatriphosphinan-2,4,6-trioxid

In einem Glaskolben mit Rührer, Stopfen, Innenthermometer und Destillationskolonne werden, unter Argon, 33,0 g Propanphosphonsäure (0,27 mol) in 189,3 g Essigsäureanhydrid (1,85 mol) gelöst und 2h zum Rückfluß erhitzt. Anschließend wird bei 100 mbar das Gemisch aus Essigsäure und Essigsäureanhydrid abdestilliert. Die Außentemperatur wird auf 350°C und das Vakuum auf 0,1 mbar erhöht. Bei 280°C Kopftemperatur fallen 22,9 g farbloses, siropöses 2,4,6-Tripropyl-[1,3,5,2,4,6]-trioxatriphosphinan-2,4,6-trioxid an (Ausbeute 80 %).
Das so erhaltene CPA wird in 22,9 g Dichlormethan gelöst, und kann in dieser Form zur weiteren Synthese eingesetzt werden.

### Beispiel 2: Synthese von 2,4,6-Tripropyl-[1,3,5,2,4,6]-trioxatriphosphinan-2,4,6-trioxid

In einem Glaskolben mit Rührer, Stopfen, Innenthermometer und Destillationskolonne werden, unter Argon, 33,0 g Propanphosphonsäure (0,27 mol) in 189,3 g Essigsäureanhydrid (1,85 mol) gelöst und 2 h zum Rückfluß erhitzt. Anschließend wird bei 100 mbar das Gemisch aus Essigsäure und Essigsäureanhydrid abdestilliert. Die Außentemperatur wird auf 350°C und das Vakuum auf 0,1 mbar erhöht. Bei 280 °C Kopftemperatur fallen 22,9 g farbloses, siropöses 2,4,6-Tripropyl-[1,3,5,2,4,6]-trioxatriphosphinan-2,4,6-trioxid an (Ausbeute 80 %).
Das so erhaltene CPA wird in 22,9 g Dimethylformamid gelöst, und kann in dieser Form zur weiteren Synthese eingesetzt werden.

### Beispiel 3: Synthese von 2,4,6-Triethyl-[1,3,5,2,4,6]-trioxatriphosphinan-2,4,6-trioxid

In einem Glaskolben mit Rührer, Stopfen, Innenthermometer und Destillationskolonne werden, unter Argon, 40,2 g Ethanphosphonsäure (0,37 mol) in 204,9 g Essigsäureanhydrid (2,01 mol) gelöst und 2h zum Rückfluß erhitzt.

Anschließend wird bei 100 mbar das Gemisch aus Essigsäure und Essigsäureanhydrid abdestilliert. Die Außentemperatur wird auf 350°C und das Vakuum auf 0,1 mbar erhöht. Bei 295 °C Kopftemperatur fallen 20,2 g farbloses, siropöses 2,4,6-Triethyl-[1,3,5,2,4,6]-trioxatriphosphinan-2,4,6-trioxid an (Ausbeute 66 %).

### Beispiel 4: Synthese von 2,4,6-Trihexyl-[1,3,5,2,4,6]-trioxatriphosphinan-2,4,6-trioxid

In einem Glaskolben mit Rührer, Stopfen, Innenthermometer und Destillationskolonne werden, unter Argon, 44,8 g Hexanphosphonsäure (0,27 mol) in 189,3 g Essigsäureanhydrid (1,85 mol) gelöst und 2 h zum Rückfluß erhitzt. Anschließend wird bei 100 mbar das Gemisch aus Essigsäure und Essigsäureanhydrid abdestilliert. Die Außentemperatur wird auf 350°C und das Vakuum auf 0,1 mbar erhöht. Bei 240°C Kopftemperatur fallen 30,0 g farbloses, siropöses 2,4,6-Trihexyl-[1,3,5,2,4,6]-trioxatriphosphinan-2,4,6-trioxid an (Ausbeute 75 %).
Das so erhaltene CPA wird in 30 g Dichlormethan gelöst, und kann in dieser Form zur weiteren Synthese eingesetzt werden.

### Beispiel 5: Synthese von 2,4,6-Tricyclohexyl-[1,3,5,2,4,6]-trioxatriphosphinan-2,4,6-trioxid

In einem Glaskolben mit Rührer, Stopfen, Innenthermometer und Destillationskolonne werden, unter Argon, 44,3 g Cyclo-Hexanphosphonsäure (0,27 mol) in 189,3 g Essigsäureanhydrid (1,85 mol) gelöst und 2 h zum Rückfluß erhitzt. Anschließend wird bei 100 mbar das Gemisch aus Essigsäure und Essigsäureanhydrid abdestilliert. Die Außentemperatur wird auf 350°C und das Vakuum auf 0,1 mbar erhöht. Bei 260°C Kopftemperatur fallen 27,6 g farbloses, siropöses 2,4,6-Tricyclohexyl-[1,3,5,2,4,6]-trioxatriphosphinan-2,4,6-trioxid an (Ausbeute 70 %).
Das so erhaltene CPA wird in 27,6 g Dichlormethan gelöst, und kann in dieser Form zur weiteren Synthese eingesetzt werden.

### Beispiel 6: Synthese von N-Acetyl-L-phenylalaninyl-L-alaninmethylester

0,1 g L-Alaninmethylesterhydrochlorid (0,7 mmol), 0,2 g N-Acetyl-L-phenylalanin (1 mmol) und 0,55 ml N-Methylmorpholin (5 mmol) werden in 50 ml Dichlormethan gelöst und auf -10°C abgekühlt. Es werden langsam 0,5 g CPA aus Beispiel 1 (50 % in CH₂Cl₂; 0,8 mmol) zugegeben, 3 h in der Kälte und 72 h bei Raumtemperatur gerührt. Die Lösung wird eingedampft und mit Essigester und 1 N HCl-Lösung, ges. NaHCO₃, ges. NaCl und dest. Wasser extrahiert. Die organische Phase wird über Magnesiumsulfat getrocknet, abfiltriert und eingedampft. Es fallen 0,18 g des weißen N-Acetyl-L-phenylalaninyl-L-alaninmethylester an (88 %).

### Beispiel 7: Synthese von N-Acetyl-L-phenylalaninyl-L-phenylalaninmethylester

0,38 g L-Phenylalaninmethylesterhydrochlorid (1,8 mmol), 0,37g N-Acetyl-L-Phenylalanin (1,7 mmol) und 1,6 ml N-Methylmorpholin (14,6 mmol) werden in 30 ml Dimethylacetamid gelöst und auf 0°C abgekühlt. Nun werden 1,2 ml CPA aus Beispiel 2 (50% in Dimethylformamid, 1,9 mmol) zugegeben. Es wird noch 1 h bei 0°C und 12 h bei Raumtemperatur gerührt. Die Lösung wird eingedampft und mit Essigester und 1N HCl-Lösung, ges. NaHCO₃, ges. NaCl und dest. Wasser extrahiert. Die organische Phase wird über Magnesiumsulfat getrocknet, abfiltriert und eingedampft. Es fallen 0,53 g des weißen N-Acetyl-L-phenylalaninyl-L-phenylalaninmethylester an (84 %).

### Vergleichsbeispiel 8: Synthese von N-Acetyl-L-phenylalaninyl-L-phenylalaninmethylester mit gleicher Menge OPA wie in Beispiel 5

0,38 g L-Phenylalaninmethylesterhydrochlorid (1,8 mmol), 0,37 g N-Acetyl-L-Phenylalanin (1,7 mmol) und 1,6 ml N-Methylmorpholin (14,6 mmol) werden in 30 ml Dimethylacetamid gelöst und auf 0°C abgekühlt. Nun werden 1,2 ml OPAhergestellt wie beschrieben in EP 0 527 442, Beispiel 1 - (50 % in Dimethylformamid, Mol-Angabe nicht möglich, weil die Zusammensetzung nicht bekannt ist) zugegeben. Es wird noch 1 h bei 0°C und 12 h bei Raumtemperatur gerührt. Die Lösung wird eingedampft und mit Essigester und 1 N HCl-Lösung, ges. NaHCO₃, ges. NaCl und dest. Wasser extrahiert. Die organische Phase wird über Magnesiumsulfat getrocknet, abfiltriert und eingedampft. Es fallen 0,14 g des weißen N-Acetyl-L-phenylalaninyl-L-phenylalaninmethylester an (22 %).

### Vergleichsbeispiel 9: Synthese von N-Acetyl-L-phenylalaninyl-L-phenylalaninmethylester mit einer erhöhten Mege an OPA im Vergleich zu Beispiel 5

0,38 g L-Phenylalaninmethylesterhydrochlorid (1,8 mmol), 0,37 g N-Acetyl-L-Phenylalanin (1,7 mmol) und 5,0 ml N-Methylmorpholin (45,5 mmol) werden in 30 ml Dimethylacetamid gelöst und auf 0°C abgekühlt. Nun werden 5 ml OPA - hergestellt wie beschrieben in EP 0 527 442, Beispiel 1 - (50 % in Dimethylformamid, Mol-Angabe nicht möglich, weil die Zusammensetzung nicht bekannt ist) zugegeben. Es wird noch 1 h bei 0°C und 12 h bei Raumtemperatur gerührt. Die Lösung wird eingedampft und mit Essigester und 1N HCl-Lösung, ges. NaHCO₃, ges. NaCl und dest. Wasser extrahiert. Die organische Phase wird über Magnesiumsulfat getrocknet, abfiltriert und eingedampft. Es fallen 0,55 g des weißen N-Acetyl-L-phenylalaninyl-L-phenylalaninmethylester an (87 %).

## Patentansprüche

1. Verfahren zur Herstellung von cyclischen Phosphonsäureanhydriden der Formel (III) durch
a) Umsetzung von Phosphonsäure-Derivaten der Formel (I) mit Essigsäureanhydrid bei einer Temperatur im Bereich von 30 bis 150 °C und gleichzeitiger destillativer Abtrennung eines Gemisches aus Essigsäure und Essigsäureanhydrid
und
b) anschließende Reaktivdestillation der in Schritt a) erhaltenen oligomeren Phosphonsäureanhydride der Formel (II) und Überführung in die entsprechenden cyclischen trimeren Phosphonsäureanhydride der Formel (III) 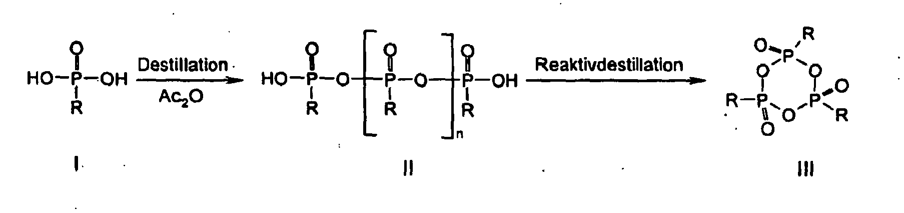
wobei
n eine ganze Zahl von 0 bis 300 ist und
R für H, Fluor, Chlor, Brom, lod, Allyl, Aryl oder offenkettige cyclische oder verzweigte C₁- bis C₈-Alkyl-Reste, Aryloxy, Allyloxy oder Alkoxy mit offenkettigen cyclischen oder verzweigten C₁- bis C₈-Alkyl-Resten, Nitro, Nitril, Carboxy, Carbonsäureester mit offenkettigen oder verzweigten C₁ bis C₈-Alkyl-Resten, Amid- oder Alkylamidreste mit offenkettigen oder verzweigten C₁ bis C₈-Alkyl-Resten, steht und
wobei
die in Schritt b) gebildeteten cyclischen trimeren Phosphonsäureanhydride sofort in einem organischen Lösungsmittel gelöst werden, das sich diesen gegenüber inert verhält.

2. Verfahren nach Anspruch 1, **dadurch gekennzeichnet, dass** das Verhältnis von Essigsäureanhydrid zu Phosphonsäure der Formel (I) im Bereich von 20 : 1 und 1 : 1 liegt.

3. Verfahren nach Anspruch 1 oder 2, **dadurch gekennzeichnet, dass** die Reaktivdestillation in Schritt b) bei einer Temperatur im Bereich von 100 bis 450 °C (Reaktorinnentemperatur) und einer Kopftemperatur von 100 bis 380 °C erfolgt.

4. Verfahren nach mindestens einem der Ansprüche 1 bis 3, **dadurch gekennzeichnet, dass** der Druck
a) bei der Destillation von Essigsäure und unumgesetztem Essigsäureanhydrid zwischen 1 mbar und 1000 mbar und
b) bei der Reaktivdestillation der oligomeren Phosphonsäureanhydride der Formel (II) zu den cyclischen Phosphonsäureanhydriden der Formel (III) in einem Druckbereich zwischen 0,001 mbar und 500 mbar
liegt.

5. Verfahren nach mindestens einem der Ansprüche 1 bis 4, **dadurch gekennzeichnet, dass** das Verfahren kontinuierlich durchgeführt wird.

6. Verfahren nach mindestens einem der Ansprüche 1 bis 5, **dadurch gekennzeichnet, dass** die erhaltenen cyclischen trimeren Phosphonsäureanhydride der Formel (III) nach der Reaktivdestillation in einem organischen Lösungsmittel gelöst werden in einem Mischungsverhältnis von Lösungsmittel zu Phosphonsäureanhydrid im Bereich von 10 : 1 und 1 : 10.

7. Verfahren nach mindestens einem der Ansprüche 1 bis 6, **dadurch gekennzeichnet, dass** das organische Lösungsmittel Ligroin, Sulfolan, DMSO, HMPT, NMP, Pentan, Hexan, Heptan, Octan, Cyclopentan, Cyclohexan, Cycloheptan, Cyclooctan, Dichlormethan, Chloroform, Tetrachlorkohlenstoff, 1,2-Dichlorethan, 1,1,2,2-Tetrachlorethan, Methylacetat, Ethylacetat, Propylacetat, Buthylacetat, Dimethylformamid, Diethylformamid, Dimethylacetamid, Diethylacetamid, Diethylether, Diisopropylether, tert-Buthyl-Methylether, THF, Dioxan, Acetonitril, oder ein Gemisch derselben ist.

## Claims

1. A process for preparing cyclic phosphonic anhydrides of the formula (III) by
a) reaction of phosphonic acid derivatives of the formula (I) with acetic anhydride at a temperature in the range from 30 to 150°C and simultaneous distillative removal of a mixture of acetic acid and acetic anhydride,
and
b) subsequent reactive distillation of the oligomeric phosphonic anhydrides of the formula (II) obtained in step a) and conversion to the corresponding cyclic trimeric phosphonic anhydrides of the formula (III) 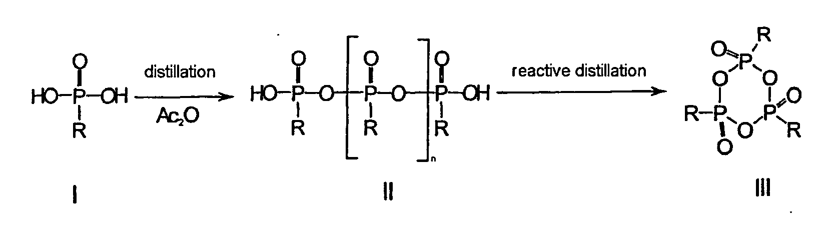
where
n is an integer from 0 to 300 and
R are H, fluorine, chlorine, bromine, iodine, allyl, aryl or open-chain cyclic or branched C₁ to C₈-alkyl radicals, aryloxy, allyloxy or alkoxy having open-chain cyclic or branched C₁ to C₈-alkyl radicals, nitro, nitrile, carboxyl, carboxylic esters having open-chain or branched C₁ to C₈-alkyl radicals, amide or alkylamide radicals having open-chain or branched C₁ to C₈-alkyl radicals, and
the cyclic trimeric phosphonic anhydrides formed in step b) being immediately dissolved in an organic solvent which is inert toward them.

2. The process as claimed in claim 1, wherein the ratio of acetic anhydride to phosphonic acid of the formula (I) is in the range from 20:1 to 1:1.

3. The process as claimed in claim 1 or 2, wherein the reactive distillation in step b) is effected at a temperature in the range from 100 to 450°C (the internal reactor temperature) and a top temperature of from 100 to 380°C.

4. The process as claimed in at least one of claims 1 to 3, wherein the pressure
a) in the distillation of acetic acid and unconverted acetic anhydride is between 1 mbar and 1000 mbar, and
b) in the reactive distillation of the oligomeric phosphonic anhydrides of the formula (II) to give the cyclic phosphonic anhydrides of the formula (III) is within a pressure range between 0.001 mbar and 500 mbar.

5. The process as claimed in at least one of claims 1 to 4, which is carried out continuously.

6. The process as claimed in at least one of claims 1 to 5, wherein the resulting cyclic trimeric phosphonic anhydrides of the formula (III), after the reactive distillation, are dissolved in an organic solvent in a mixing ratio of solvent to phosphonic anhydride in the range from 10:1 to 1:10.

7. The process as claimed in at least one of claims 1 to 6, wherein the organic solvent is ligroin, sulfolane, DMSO, HMPT, NMP, pentane, hexane, heptane, octane, cyclopentane, cyclohexane, cycloheptane, cyclooctane, dichloromethane, chloroform, carbon tetrachloride, 1,2-dichloroethane, 1,1,2,2-tetrachloroethane, methyl acetate, ethyl acetate, propyl acetate, butyl acetate, dimethylformamide, diethylformamide, dimethylacetamide, diethylacetamide, diethyl ether, diisopropyl ether, tert-butyl methyl ether, THF, dioxane, acetonitrile or a mixture thereof.

## Revendications

1. Procédé de fabrication d'anhydrides d'acide phosphonique cycliques de formule (III) par
a) réaction de dérivés d'acide phosphonique de formule (I) avec un anhydride d'acide acétique à une température dans la plage de 30 à 150°C et séparation simultanée par distillation d'un mélange d'acide acétique et d'anhydride d'acide acétique
et
b) distillation réactive consécutive des oligomères d'anhydrides d'acide phosphonique de formule (II), obtenus à l'étape a), et transformation en trimères d'anhydrides d'acide phosphonique cycliques correspondants de formule (III) : 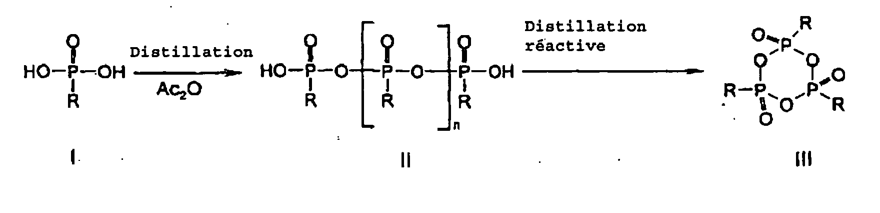
dans laquelle :
n est un nombre entier de 0 à 300, et
R représente l'hydrogène, le fluor, le chlore, le brome, l'iode, des groupements allyle, aryle ou des radicaux alkyle en C₁-C₈ cycliques ou ramifiés à chaîne ouverte, des groupements aryloxy, allyloxy ou alcoxy avec des radicaux alkyle en C₁-C₈ cycliques ou ramifiés à chaîne ouverte, des groupements nitro, nitrile, carboxyle, ester d'acide carboxylique avec des radicaux alkyle en C₁-C₈ à chaîne ouverte ou ramifiés, des radicaux amide ou alkylamide avec des radicaux alkyle en C₁-C₈ à chaîne ouverte ou ramifiés, et
dans lequel procédé :
les trimères d'anhydrides d'acide phosphonique cycliques, formés à l'étape b), sont immédiatement dissous dans un solvant organique, qui a un comportement inerte vis-à-vis de ceux-ci.

2. Procédé selon la revendication 1, **caractérisé en ce que** le rapport de l'anhydride d'acide acétique à l'acide phosphonique de formule (I) se situe dans la plage de 20:1 à 1:1.

3. Procédé selon la revendication 1 ou 2, **caractérisé en ce que** la distillation réactive à l'étape b) s'effectue à une température dans la plage de 100 à 450 C (température interne du réacteur) et à une température de tête de 100 à 380°C.

4. Procédé selon au moins l'une quelconque des revendications 1 à 3, **caractérisé en ce que** la pression se situe
a) entre 1 mbar et 1000 mbars lors de la distillation de l'acide acétique et de l'anhydride d'acide acétique qui n'a pas réagi, et
b) dans une plage de pression entre 0,001 mbar et 500 mbars lors de la distillation réactive des oligomères d'anhydrides d'acide phosphonique de formule (II) en anhydrides d'acide phosphonique cycliques de formule (III).

5. Procédé selon au moins l'une quelconque des revendications 1 à 4, **caractérisé en ce que** le procédé est réalisé en mode continu.

6. Procédé selon au moins l'une quelconque des revendications 1 à 5, **caractérisé en ce que** les trimères d'anhydrides d'acide phosphonique cycliques de formule (III) obtenus sont dissous après la distillation réactive dans un solvant organique dans un rapport de mélange du solvant à l'anhydride d'acide phosphonique situé dans la plage de 10 : 1 à 1 : 10.

7. Procédé selon au moins l'une quelconque des revendications 1 à 6, **caractérisé en ce que** le solvant organique est la ligroïne, le sulfolane, le DMSO, le HMPT, la NMP, le pentane, l'hexane, l'heptane, l'octane, le cyclopentane, le cyclohexane, le cycloheptane, le cyclooctane, le dichlorométhane, le chloroforme, le tétrachlorure de carbone, le 1,2-dichloréthane, le 1,1,2,2-tétrachloréthane, l'acétate de méthyle, l'acétate d'éthyle, l'acétate de propyle, l'acétate de butyle, le diméthylformamide, le diéthylformamide, le diméthylacétamide, le diéthylacétamide, le diéthyléther, le diisopropyléther, le tert-butylméthyléther, le THF, le dioxane, l'acétonitrile, ou un mélange de ceux-ci.
